# EUROPÄISCHE PATENTSCHRIFT

(11) **EP 0 873 114 B1**
(45) Veröffentlichungstag und Bekanntmachung des Hinweises auf die Patenterteilung: **04.12.2002**
(21) Anmeldenummer: 97900560.0
(22) Anmeldetag: 03.01.1997
(51) Int. Cl.: A61K 9/16, A61K 47/32

(54) **GRANULATE FÜR KOSMETISCHE UND PHARMAZEUTISCHE ZUBEREITUNGEN**
GRANULATES FOR COSMETIC AND PHARMACEUTICAL PREPARATIONS
GRANULES POUR PREPARATIONS COSMETIQUES ET PHARMACEUTIQUES

(30) Priorität: 08.01.1996 DE 19600324
(43) Veröffentlichungstag der Anmeldung: 28.10.1998
(73) Patentinhaber: BASF AKTIENGESELLSCHAFT, 67056 Ludwigshafen (DE)
(72) Erfinder: SCHADE, Christian, D-67061 Ludwigshafen (DE); HEINZ, Robert, D-67067 Ludwigshafen (DE); BÖRS, Thekla, D-80687 München (DE); WEKEL, Hans-Ulrich, D-67158 Ellerstadt (DE)
(86) Internationale Anmeldenummer: EP9700014
(87) Internationale Veröffentlichungsnummer: WO97025027

(56) Entgegenhaltungen:
- EP-A- 0 376 891
- EP-A- 0 514 008
- EP-A- 0 516 141
- US-A- 3 459 850
- US-A- 5 169 645

## Beschreibung

Die Erfindung betrifft Granulate, erhältlich durch Verpressen von Mischungen aus mindestens einem pulverförmigen, rheologiemodifizierenden, carboxylgruppenhaltigen Polymer als Hauptkomponente und mindestens einer öllöslichen Komponente.

Es ist bekannt, daß schwach vernetzte, ggf. hydrophobierte Polycarbonsäuren in der Kosmetik, der Medizin und der Pharmazie breit angewendet werden. Solche Polymere werden beispielsweise unter den Handelsnamen Carbopol®, Pemulen®, Synthalen®, Rheolate®, Stabylen®, Acrisint®, Junlon®, Hypan® oder Hivis® auf dem Markt angeboten.

Diese Produkte sind feinteilige, oft elektrostatisch leicht aufladbare und stark staubende Pulver. Der hohe Feinanteil dieser Pulver führt zu einer Reihe von Nachteilen bei der Verarbeitung wie etwa die leichte Entmischung der verschiedenen großen Partikel, Agglomeratbildung, ungünstiges Fließverhalten und Materialverluste durch Feinstaub. Dieser Feinstaub birgt zudem gesundheitliche Gefahren, da ein nennenswerter Anteil dieses Staubes Teilchendurchmesser von weniger als 5 µm besitzt und daher lungengängig ist. Bei der Verarbeitung dieser Polymere sind deshalb besondere Sicherheits- und Arbeitshygienemaßnahmen erforderlich.

Durch Preßagglomeration läßt sich die Teilchengröße von Polymeren erhöhen und damit die Handhabung der Pulver verbessern. In Chem. Ing.-Tech. 59 (1987) Nr. 10: 779-787, Chemie-Technik, Vol. 4, (1975) Nr: 6: 207-209, und Aufbereitungs-Technik Nr. 10 (1980): 525-533 werden die Prinzipien der Preßagglomeration am Beispiel von Düngemitteln beschrieben. Für eine gute Preßagglomeration sind niedrige Feuchtigkeitsgehalte des zu pressenden Materials vorteilhaft. Für die Kompaktierung ist es typisch, daß im Prozeßverlauf weder ein Zusatz an Feuchtigkeit noch an Bindemittel erfolgt. Die bindemittellose Preßagglomeration läßt unter hohen Drücken ausschließlich Bindemechanismen innerhalb des Polymers wirksam werden.

US 5,169,645 beschreibt die Herstellung Wachs enthaltender Granulate durch Umsetzung des Wachses mit einem Polymer aus vernetzten Acrylsäurehomopolymeren in der Schmelze. Diese Granulate werden für die Herstellung oraler oder anderer pharmazeutischer Darreichungsformen verwendet.

In EP-A-0 514 008 wird ein Wirkstoff-Applikationssystem mit kontrollierter Freigabe des Wirkstoffs an die Gastrointestinale Mucosa beschrieben. Dieses Applikationssystem besteht neben dem Wirkstoff aus einem Viskosität modifizierenden Stoff wie einem Acrylsäure-/Stearylmethacrylat Copolymer und einem Polyglycerinfettsäureester oder Lipid.

EP-A-0 516 141 offenbart eine pharmazeutische Zusammensetzung zur kontrollierten Freigabe eines Wirkstoffs in Form von bioadhäsiven Granulaten, die aus einem Wirkstoff und einer Komponente, die die Freisetzung des Wirkstoffes am biologischen Gewebe kontrollieren, und einem bioadhäsiven Überzug bestehen, der aus einem Polymer besteht.

Auch von EP-A-0 376 891 wird eine pharmazeutische Zusammensetzung für die Applikation des Wirkstoffs Baclofen in Form einer Klebetablette offenbart. Diese Klebetablette besteht aus einem hydrophilen Tablettenkern, der an der Mundschleimhaut haftet und den Wirkstoff Baclofen, ein quellfähiges Vinylpolymerisat, ein Galactomannan und/oder ein pharmazeutisch verwendbares Wachs enthält, und einem hydrophoben Überzug.

US 3,459,850 offenbart eine pharmazeutische Zubereitung mit kontrollierter Freigabe in Form einer Tablette. Diese Tablette wird durch Mischen eines Wirkstoffes mit einem verdaubaren Fett, einem unverdaubaren Wachs und einem Polymer mit anschließender Tablettierung hergestellt.

Falls das zu granulierende Material unter dem Druck der Granulierwerkzeuge nicht die notwendigen Bindeeigenschaften aufweist, können in Ausnahmefällen feste oder flüssige Komponenten zur Bindungsverbesserung zugesetzt werden.

Die Preßagglomeration von Polymeren wird beispielsweise in Eur. J. Pharm. Biopharm 38 (6) 195-198 (1992) oder in der WO 93/23457 beschrieben. Diese Produkte eignen sich zur Herstellung wäßriger Gele, sind jedoch für die Herstellung ölhaltiger Systeme ungeeignet, da sie Gelbrocken bilden. Bei der Herstellung ölhaltiger Systeme wie beispielsweise von Emulsionen ist es üblich, die Polymere zunächst in der Ölphase zu dispergieren und anschließend weitere Komponenten, wie z.B. Wasser und/oder eine Base, zuzusetzen. Die nach dem Stand der Technik hergestellten Preßagglomerate zerfallen unter den gegebenen Bedingungen nicht vollständig in der ölphase, so daß bei der nachfolgenden Zugabe von Wasser und/oder einer Base stark nachquellende, klumpige, große Gelbrocken enthaltende Mischungen entstehen, die für den kosmetischen und pharmazeutischen Gebrauch nicht verwendungsfähig sind.

Es bestand daher die Aufgabe, staubarme, rieselfähige Granulate von pulverförmigen Polymeren, die sich ohne die beschriebenen Nachteile in Ölphasen einarbeiten lassen, zu entwickeln und für Anwendungen im kosmetischen und pharmazeutischen Bereich zur Verfügung zu stellen.

Demgemäß wurde nun gefunden, daß bei den erfindungsgemäßen Granulaten, erhältlich durch Verpressen von Mischungen aus mindestens einem pulverförmigen, rheologiemodifizierenden, carboxylgruppenhaltigen Polymer als Hauptkomponente und mindestens einer öllöslichen Komponente, wobei eine Polymerkomponente erhältlich durch radikalisch initiierte Copolymerisation von Monomergemischen aus
a) 50 bis 99,93 Gew.-% einer monoethylenisch ungesättigten C₃-C₆-Monocarbonsäure, einer monoethylenisch ungesättigten C₄-C₈-Dicarbonsäure oder deren Anhydride oder Salze oder Mischungen aus den genannten Carbonsäuren, deren Anhydriden und/oder Salzen,
b) 0,05 bis 5 Gew.-% einer oder mehrerer Verbindungen mit mindestens zwei ethylenisch ungesättigten, nicht konjugierten Doppelbindungen im Molekül als Vernetzer,
c) 0,02 bis 10 Gew.-% mindestens eines C₁-C₃₀-Alkyl(meth)acrylats und
d) 0 bis 50 Gew.-% anderen mit den Monomeren (a), (b) und (c) copolymerisierbaren wasserunlöslichen Monomeren verwendet wurde;
durch Zugabe mindestens einer öllöslichen Komponente zum pulverförmigen Polymer vor oder während der Preßagglomeration ein Granulat entsteht, das sich exzellent in Öl dispergieren läßt und darüber hinaus ohne aufwendige Sicherheitsmaßnahmen handhabbar ist.

Die erfindungsgemäßen Granulate lösen sich ohne Bildung von Gelbrocken auf und bilden so homogene Emulsionen. Daher sind sie ausgezeichnet für die Herstellung von pharmazeutischen und insbesondere kosmetischen Zubereitungen auf Basis von Öl-in-Wasser-Emulsionen geeignet. Gegenstand der Erfindung ist daher, diese Verwendung der oben beschriebenen Granulate und entsprechende Zubereitungen.

Granulierbare carboxylgruppenhaltige Polymere sind im Handel eingeführt oder in der Literatur beschrieben. Beispielsweise findet sich eine Aufstellung von in der Kosmetik handelsüblichen Produkten in D. Laba (Hrsg.), Rheological Properties of Cosmetics and Toiletries, M. Dekker Inc., 1993, Seite 55 bis 152. Bevorzugt eingesetzt werden Polycarbonsäuren und Polycarbonsäurecopolymerisate.

Diese schwach vernetzten ggf. hydrophobierten Polycarbonsäuren werden bevorzugt nach dem Verfahren der Fällungspolymerisation in organischen Medien hergestellt. In DE-A-43 25 158 wird die Fällungspolymerisation beschrieben. Beispiele für solche Polymerisate geben die Patentschriften EP-A-128 237, EP-A-584 771, EP-A-371 421, EP-A-470 098, US 4 066 583, US 3 915 921, US 2 798 053, US 5 034 486, US 5 034 488, WO 92/01724, EP-A-238 404, EP-A-436 960, DE-A-4 213 283, DE-A-4 213 971 oder DE-A-4 325 158 wieder.

Besonders vorteilhaft werden erfindungsgemäß pulverförmige, rheologiemodifizierende carboxylgruppenhaltige Polymere, erhältlich durch radikalisch initiierte Copolymerisation von Monomergemischen aus
a) 50 bis 99,93 Gew.-% einer monoethylenisch ungesättigten C₃-C₆-Monocarbonsäure, einer monoethylenisch ungesättigten C₄-C₈-Dicarbonsäure oder deren Anhydride oder Salze oder Mischungen aus den genannten Carbonsäuren, deren Anhydriden und/oder Salzen,
b) 0,05 bis 5 Gew.-% einer oder mehrerer Verbindungen mit mindestens zwei ethylenisch ungesättigten, nicht konjugierten Doppelbindungen im Molekül als Vernetzer,
c) 0,02 bis 10 Gew.-% mindestens eines C₁-C₃₀-Alkyl(meth)acrylats und
d) 0 bis 50 Gew.-% anderen mit den Monomeren (a), (b) und (c) copolymerisierbaren wasserunlöslichen Monomeren, granuliert,
wobei die verschiedenen Monomere sich zu 100 % addieren.

Die Monomere a) sind monoethylenisch ungesättigte C₃-C₈-Mono- und Dicarbonsäuren, ihre Anhydride, ihre Salze oder Mischungen aus den genannten Carbonsäuren, Anhydriden und Salzen. Geeignete Carbonsäuren sind beispielsweise Acrylsäure, Methacrylsäure, Maleinsäure, Fumarsäure, Itaconsäure oder Crotonsäure. Als Anhydride kommen beispielsweise Acrylsäureanhydrid, Methacrylsäureanhydrid oder Maleinsäureanhydrid in Betracht. Von den Monomeren der Gruppe a) werden Acrylsäure, Methacrylsäure, Maleinsäure und/oder Maleinsäureanhydrid bevorzugt eingesetzt. Die Monomeren können in den zur Polymerisation vorgesehenen Monomermischungen beispielsweise zu 50 bis 99,93 Gew.-% enthalten sein. Ist das Monomer a) Fumarsäure, Maleinsäure oder Maleinsäureanhydrid, wird es besonders bevorzugt in Mengen von 50 - 80 %, ganz besonders bevorzugt in Mengen von 50 - 64,8 Gew.-% eingesetzt. Ist das Monomer a) Acryl- oder Methacrylsäure, wird es besonders bevorzugt in Mengen von 70 - 99,93 %, ganz besonders bevorzugt'in Mengen von 85 - 99,8 Gew.-% eingesetzt.

Die Monomeren a) können auch teilweise in Form ihrer Salze vorliegend eingebaut sein. Dies kann beispielsweise durch Zusatz mindestens einer Base vor, während oder nach der Polymerisation erreicht werden. Sofern die Polymere in Form ihrer Salze eingesetzt werden, sind bis zu 90 mol-%, vorzugsweise bis zu 40 mol-% der Carboxylfunktionen umgesetzt. Im besonders bevorzugten Fall liegen die Carboxylgruppen jedoch zu über 90 mol-% in freier Form oder als Anhydrid gebunden vor.

Liegen die Monomere in Form ihrer Salze vor, so sind die Erdalkali-, Alkali- oder Ammoniumsalze oder die Salze organischer Amine bevorzugt, besonders bevorzugt sind die Alkali- oder Ammoniumsalze.

Als Vernetzer wurden zu diesem Zweck allgemein bekannte Verbindungen verwendet, die mindestens 2 monoethylenisch ungesättigte Gruppen im Molekül enthalten.

Geeignete Vernetzer dieser Art sind zum Beispiel Acrylester, Methacrylester, Allylether oder Vinylether von mindestens zweiwertigen Alkoholen. Die OH-Gruppen der zugrundeliegenden Alkohole können dabei ganz oder teilweise verethert oder verestert sein. Beispielsweise finden zweiwertige Alkohole Verwendung wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglycol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäureneopentylglycolmonoester, 2,2-Bis(4-hydroxyphenyl)propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thiopentan-1,5-diol, sowie Polyethylenglykole, Polypropylenglykole und Polytetrahydrofurane mit mittleren Molekulargewichten von jeweils 200 bis 10 000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden.

Weitere geeignete Vernetzer sind die Vinylester oder die Ester einwertiger, ungesättigter Alkohole mit ethylenisch ungesättigten C₃- bis C₆-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäure verestern, beispielsweise Malonsäure, Weinsäure, Trimellitsäure, Phthalsäure, Terephthalsäure, Citronensäure oder Bernsteinsäure.

Weitere geeignete Vernetzer sind Ester ungesättigter Carbonsäuren mit den oben beschriebenen mehrwertigen Alkoholen, beispielsweise der Ölsäure, Crotonsäure, Zimtsäure oder 10-Undecensäure.

Geeignet sind außerdem geradkettig oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z.B. Divinylbenzol, Divinyltoluol, 1,7-Octadien, 1,9-Decadien, 4-Vinyl-1-cyclohexen, Trivinylcyclohexan oder Polybutadiene mit mittleren Molekulargewichten von 200 - 20 000. Als Vernetzer sind ferner geeignet mindestens zwei Doppelbindungen enthaltende Acrylsäureamide und Methacrylsäureamide von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Geeignet sind auch N-Allyl- oder N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid.

Weitere geeignete Vernetzer sind Divinyldioxan, Diallylphosphat, Triallylphosphat, Triallyltriazintrion, Diallylamin, Triallylamin, Tetraallylsilan, Tetravinylsilan, Hexaallyltrimethylentrisulfon, Butadien oder Isopren. Selbstverständlich können auch Mischungen der vorgenannten Verbindungen eingesetzt werden.

Bevorzugt eingesetzt werden (Meth)acrylester von C₂-C₆-Diolen, Allylether von mehrwertigen Alkoholen wie z.B. von Trimethylolpropan, Penaterythrit, Saccharose oder Sorbit mit mehr als einer Allyletherfunktion im Molekül, (Meth)acrylsäureester von ungesättigten C₃-C₂₄-Alkoholen, z.B. Oleyl(meth)acrylat, Trivinylcyclohexan und/oder nicht-konjugierte C₆-C₁₆-Alkadiene sowie Divinylglycol. Ganz besonders bevorzugt eingesetzt werden Allylether von Penaterythrit oder Saccharose mit mehr als zwei Allyletherfunktionen im Molekül, Allyl(meth)acrylat, (Meth)acrylsäureester von ungesättigten C₁₂-C₂₀-Alkoholen, Trivinylcyclohexan und/oder nicht-konjugierte C₈-C₁₄-Alkadiene. Sofern vernetzende Monomere b) zugegen sind, können sie in Mengen von 0,05 bis 5 Gew.-% eingesetzt werden. Vorzugsweise verwendet man 0,1 bis 3 Gew.-% der Monomere b).

Monomere der Gruppe c) sind Acrylester oder Methacrylester von gesättigten, linearen oder verzweigten C₁-C₄₀-Alkoholen. Beispiele sind Methyl-, Ethyl-, n-Propyl-, i-Propyl-, n-Butyl-, i-Butyl-, tert.-Butyl-, n-Hexyl-, n-Octyl-, i-Nonyl-, n-Decyl-, n-Dodecyl-, n-Tetradecyl, n-Hexadecyl-, n-Heptadecyl-, n-Octadecyl-, n-Nonadecyl-, n-Eicosyl-, n-Docosyl-, n-Tetracosyl-, 2-Ethylhexyl-, i-Bornylacrylat oder Cyclohexylacrylat bzw. die entsprechenden Methacrylate. Bevorzugt eingesetzt werden C₁-, C₂- und/oder C₆-C₃₀-Alkylacrylate oder -methacrylate eingesetzt. Besonders bevorzugt werden C₈-C₂₂-Alkylacrylate oder -methacrylate eingesetzt. Selbstverständlich können auch Mischungen der Monomere c) eingesetzt werden. Die Monomere c) sind in Mengen von 0,02 bis 10 Gew.-%, bevorzugt von 0,1 bis 5 Gew.-% zugegen.

Weitere monoethylenisch ungesättigte Monomere d) können in der Monomermischung von 0 bis 50 Gew.-% zugegen sein. Bevorzugte Monomere sind beispielsweise N-Vinylverbindungen wie N-Vinylimidazol, N-Vinylcaprolactam, 1-Vinyl-3-alkyl-imidazolium-Salze mit 8 bis 30 C-Atomen in der Alkylkette, Acrylamid, Methacrylamid, N-(C₁-C₁₈-Alkyl)acrylamide oder -methacrylamide wie N,N-Dimethylacrylamid, N-tert.-Butylacrylamid, N-tert.-Octylacryamid, N-Dodecylacrylamid, N-Methylundecylacrylamid oder N-Stearylacrylamid, Vinylester von gesättigten C₁- bis C₃₀-Carbonsäuren wie Vinylacetat, Vinylpropionat, Vinylneononanoat, Vinylneodecanoat oder Vinyllaurat, Hydroxyalkylenmono(meth)acrylester mit 2 bis 6 Kohlenstoffatomen in der Alkylengruppe, (Meth)Acrylester von mit 2 bis 50 Ethylenoxydeinheiten umgesetzten C₁-C₃₀-Alkoholen, C₁-C₃₀-Alkylvinylether, geradkettig oder verzweigte, lineare oder cyclische C₄-C₄₀-Alkene mit endständiger Doppelbindung wie Isöbuten, Diisobuten, 1-Tetracosen, 1-Docosen, 1-Eicosen, 1-Octadecen, 1-Heptadecen, 1-Hexadecen, 1-Tetradecen, 1-Dodecen, 1-Decen, Cyclododecen, Cycloocten, Styrol, α-Methylstyrol, Acrylnitril, Methylvinylketon, Vinylchlorid, Vinylidenchlorid oder Mischungen aus diesen Monomeren. Besonders bevorzugt werden Vinylacetat, Vinylmethylether, Vinylethylether oder C₄-C₃₀-Alkene. Ganz besonders bevorzugt werden Vinylmethylether oder C₁₄-C₂₂-Alkene. Falls die Monomeren zugegen sind, werden sie in einer Menge von bis zu 60 Gew.-% eingesetzt. In einem ersten Vorzugsbereich verwendet man bis zu 10 Gew.-%, insbesondere bis zu 5 Gew.-% der Monomere d), wenn a) Acryl- oder Methacrylsäure ist. In einem weiteren Vorzugsbereich wird das Monomer d) in Mengen von 20 bis 60 Gew.-%, besonders bevorzugt 35 bis 50 Gew.-% eingesetzt, wenn a) Fumarsäure, Maleinsäure oder Maleinsäureanhydrid ist. Das bevorzugte Monomer d) ist dann Vinylmethylether, Vinylethylether, Isobuten oder Diisobuten, ganz besonders bevorzugt Vinylmethylether, während das eingesetzte Monomere a) dann häufig im wesentlichen Maleinsäureanhydrid ist. In allen Fällen kann man natürlich auch Mischungen der Monomere d) verwenden.

Bei der Herstellung der Polymere aus den Monomeren a) bis d) können Additive zugegen sein, die den Polymerisationsprozeß oder die Eigenschaften des Polymerpulvers beeinflussen können. Solche Additive sind insbesondere Schutzkolloide oder Emulgatoren. Bevorzugte Emulgatoren sind oft nichtionische, niedermolekulare oder polymere, statistisch oder blockartig aufgebaute Verbindungen. Gelegentlich können solche Additive auch als Bestandteil des Polymers aufgefaßt werden, wie beispielsweise in der EP-A-584 771 und den dort zitierten Schriften beschrieben. Falls solche Additive zugegen sind, werden sie in Mengen von bis zu 20 Gew.-%, bevorzugt bis zu 10 Gew.-%, bezogen auf das Polymergewicht, eingesetzt.

Die beschriebenen Polymere können auch in Gegenwart von Zuckern, Zuckerderivaten und/oder Proteinen hergestellt werden. Beispiele für Zucker und/oder Zuckerderivate sind Glucose, Saccharose, Mannose, Stärkehydrolysate, Stärke, Cellulose oder Celluloseether. Der Anteil dieser Verbindungen im Vergleich zu Monomermischung a) bis d) kann bis zu 80 Gew.-%, vorzugsweise bis zu 50 Gew.-% betragen.

Die zur Granulatbildung vorteilhaft eingesetzten Polycarbonsäurecopolymerisate können nach verschiedenen Verfahren wie beispielsweise durch Fällungspolymerisation, umgekehrte Emulsionspolymerisation, umgekehrte Suspensionspolymerisation, Emulsionspolymerisation oder Lösungspolymerisation hergestellt werden. Sie liegen - u.U. nach weiteren Verfahrensschritten wie z.B. einer Sprühtrocknung - als feinteilige Pulver vor. Mindestens 10 Gew.-% der Teilchen des feinteiligen Polymerpulvers sollten vorzugsweise einen Teilchendurchmesser von unter 300 µm besitzen. Bevorzugt werden staubende Pulver eingesetzt, von denen über 30 Gew.-% einen Teilchendurchmesser von unter 100 µm besitzen.

Die beschriebenen Polymerpulver werden in Gegenwart mindestens einer öllöslichen Komponente zu staubarmen Granulaten verarbeitet.

Als öllösliche Komponente eignen sich viele Polymere, die vorzugsweise eine ölige oder wachsartige Konsistenz besitzen. Beispiele sind ganz oder teilweise hydrierte Polybutadiene, Polyisoprene oder ihre Mischpolymerisate mit Styrol, Polyolefinhomo- und -copolymere mit einem mittleren Molekulargewicht von weniger als 20000 oder Copolymere von N-Vinylpyrrolidon mit C₄- bis C₄₀-Alkenen.

Weiterhin geeignet sind auch Silikone, insbesondere Silikonöle, die linear, verzweigt oder cyclisch aufgebaut sein können. Eine bevorzugte Substanzklasse sind die als Cyclomethicone bezeichneten Stoffe.

Als öllösliche Komponente können auch in Wasser nicht lösliche oder schwerlösliche Lösungsmittel mit einem Siedepunkt von über 150°C verwandt werden, beispielsweise Dialkylphthalate mit 4 bis 12 Kohlenstoffatomen im Alkylrest.

Als öllösliche Gruppe eignen sich beispielsweise auch Wachse, beispielsweise pflanzliche Wachse wie Carnaubawachs, Candelillawachs, Ouricurriwachs, Zuckerrohrwachs, Retamowachs, tierische Wachse wie Bienenwachs, Schellackwachse, Insektenwachse oder Wollwachse, Erdöl-, Braunkohlen-, Torf- und andere Montanwachse, Polyolefinwachse, Paraffinwachse, Säurewachse, Esterwachse, Alkohol- oder Amidwachse. Die Wachse können naturbelassen, modifiziert, teil- oder vollsynthetisch sein. Selbstverständlich können auch einzelne Wachskomponenten wie z.B. Wachssäuren, Wachsalkohole, Wachsketone, Hydroxywachssäure, Paraffine, Harzsäuren, Polyterpene, Harzalkohole, Sterine oder Fettsäuren oder Mischungen davon eingesetzt werden.

Als öllösliche Komponente eignen sich fernerhin nichtionische Emulgatoren. Handelsübliche Emulgatoren sind beispielsweise in M. und I. Ash, Handbook of Industrial Surfactans, Gower Publishing Co., Hants, 1993 aufgeführt. Sie können niedermolekulare oder polymere Verbindungen sein. Niedermolekulare Verbindungen enthalten im allgemeinen einen geradkettig oder verzweigten, gesättigten oder ungesättigten, cyclischen oder acyclischen, aromatischen oder aliphatischen Alkylrest mit 8 bis 40, bevorzugt 10 bis 30, ganz besonders bevorzugt 12 bis 22 Kohlenstoffatomen im Molekül.

Der Alkylrest kann auch ganz oder teilweise fluoriert vorliegen. Der Alkylrest ist an einen hydrophilen Molekülteil gebunden, der mindestens ein Sauerstoff- oder Stickstoffatom enthält.

Geeignet sind auch Sterole wie Cholesterin oder Terpenoide wie Vitamin E.

Bevorzugt werden als öllösliche Komponente Öle oder Fette eingesetzt. Beispiele dafür sind Paraffinöle, Vaseline, gesättigte oder ungesättigte, geradkettige oder verzweigte C₆-C₃₀-Fettsäuren, wie Ethylhexansäure, Myristinsäure, Caprinsäure, Laurinsäure, Stearinsäure, Palmitinsäure, Isostearinsäure, Eicosansäure, Docosansäure, Tetracosansäure, Ölsäure, Linolsäure, Linolensäure, Nervonsäure, α-Hydroxynervonsäure, Elaidinsäure, Ricinolsäure, Erucasäure, Margarinsäure, Palmitoleinsäure, Stearolsäure, C₆-C₃₀-Fettsäure· oder Benzoesäureester von C₁-C₃₀-Alkoholen wie z.B. Methanol, Ethanol, iso-Propanol, Propanol, Butanol, Hexanol, Octanol, Decanol, Dodecanol, Tetradecanol, Hexadecanol, Octadecanol, 2-Ethyl-1-hexanol, Eicosanol, Docosanol, Tetracosanol, Pentaerythrit, Glycerin, Diglycerin oder Polyglycerin, natürliche Öle und Fette pflanzlichen oder tierischen Ursprungs wie Tranöle, Erdnußöl, Rapsöl, Kokosöl, Palmkernöl, Palmöl, Rüböl, Olivenöl, Leinöl, Baumwollsamenöl, Distelöl, Maisöl, Sonnenblumenöl, Sesamöl, Avocadoöl, Soyaöl, Senföl, Mandelöl, Kakaoöl, Ricinusöl, Jojobaöl, Fischöle, Talgöl, Schmalze oder Talge von Rind, Schwein oder Schaf, C₆-C₃₀-Alkohole oder durch Ethoxylierung daraus erhältliche Produkte, oder Silikonöle.

Die öllösliche Komponente ist dabei zu mindestens 2 Gew.-% in Paraffinöl, Siliconöl oder Isopropylmyristat bei Raumtemperatur löslich.

Besonders bevorzugt werden Öle eingesetzt, die auch in kosmetischen oder pharmazeutischen Formulierungen eingesetzt werden. Solche Öle werden z.B. in M. und I. Ash, Handbook of Cosmetic and Personal Care Additives, Gower Publishing Co., Aldershot, 1994 oder in H. Fiedler, Lexikon oder Hilfsstoffe, editio cantor, Aulendorf, 1989 beschrieben.

Ganz besonders bevorzugt werden Rizinusöl, Mandelöl, Paraffinöl, Finsolv®TN (C₁₂-C₁₅-Alkylbenzoat), Luvitol®EHO (2-Ethylhexansäurecetyl/stearylester), Witconol® (PPG-3-Myristylether), Miglyol®812 (Capryl-/Caprinsäuretriglycerid), Miglyol®840 (Capryl-/Caprinsäurepropylenglycoldiester), Crodamol®ISNP (Neopentansäureisostearylester), Adipinsäurediisopropylester, Myristinsäureisopropylester, Eutanol®G (2-Octyldodecanol), Cetiol®A (Laurinsäurehexylester), Cetiol®HE (Kokosfettsäure-PEG-7-Glycerylester), Cetiol®LC (Capryl-/Caprinsäureester mit gesättigten C₁₂₋₁₈-Fettalkoholen), Cetiol®SN (Isononansäurecetyl-/stearylester), Cetiol®V (Ölsäuredecylester), Amerchol®L-101 (Lanolylalkohol mit Mineralöl) oder Dow Corning®344 (Cyclomethicon) verwendet.

Im häufigsten Fall wird Paraffinöl eingesetzt.

Die öllöslichen Komponenten werden in Mengen von 0,1 bis 100 Gew.-%, bevorzugt 1 bis 50 Gew.-%, ganz besonders bevorzugt 2,5 bis 30 Gew.-%, bezogen auf das Gewicht des eingesetzten Polymerpulvers, zugesetzt.

Die beschriebenen Polymerpulver werden in Gegenwart der öllöslichen Komponente(n) mittels Preßagglomeration zu staubarmen Granulaten verarbeitet. Insbesondere eignen sich als Verfahren: die Kompaktierung mit Walzenpressen, mit anschließender Zerkleinerung, Fraktionierung und Rückführung, aber auch die Agglomeration auf anderen Pressen, z.B. durch Tablettierung.

Das Kompaktieren kann in der Weise erfolgen, daß man das Polymerpulver mittels einer Stopfschnecke in den Spalt zweier sich gegeneinander drehenden Walzen fördert und zwischen den Walzen bei Drücken in der Regel ab 10 MPa bis 300 MPa verpreßt, bevorzugt 20 - 100 MPa. Das entstehende Band ("Schülpe") wird auf einem Zerkleinerer gebrochen und z.B. mit einem Siebzerkleinerer auf die gewünschte Größe begrenzt.

Aus der entstehenden breiten Agglomeratverteilung wird anschließend mittels Sieb oder Sichter das Gut, dessen Teilchengröße kleiner als die gewünschte Untergrenze ist, abgetrennt und der Kompaktiereinrichtung zusammen mit weiterem zu kompaktierendem Pulver wieder zugeführt.

Die durch Preßagglomeration erhaltenen, staubarmen Granulate besitzen vorteilhaft mittlere Teilchengrößen im Bereich von 20 µm - 10 mm, bevorzugt 100 µm - 5 mm, ganz besonders bevorzugt 0,5 bis 3 mm.

Die so hergestellten Granulate lösen sich besonders gut in Öl auf und sind für die Herstellung von pharmazeutischen und insbesondere kosmetischen Zubereitungen auf Basis Öl-in-Wasser-Emulsionen geeignet.

Unter pharmazeutischen Zubereitungen sind z.B. Gele, Pasten, Cremen, Lotionen, Emulsionen und Salben zur Anwendung auf der gesunden, erkrankten oder verletzten Haut oder Schleimhaut zu verstehen, ohne auf diese Zubereitungen oder Anwendungsgebiete beschränkt zu sein.

Eine besonders interessante Anwendungsmöglichkeit der Granulate ist die Herstellung von transdermalen therapeutischen Systemen.

Unter kosmetischen Zubereitungen sind z.B. Hautpflegeprodukte, Hautwasch- und -reinigungsmittel, Haar- und Körperpflegemittel, Haarstylingmittel, Zahnpflegemittel, Augenpflegemittel, Fußpflegemittel, Repellent und Sonnenschutzmittel in Form von Gelen, Cremen, Lotionen, Emulsionen, Schäumen und Balsamen zu verstehen, ohne auf diese Zubereitungen oder Anwendungen beschränkt zu sein.

### Beispiele

Granulierung von Carboxylgruppen tragenden Polymeren
- Polymer 1:: Copolymer aus Acrylsäure, Stearylmethacrylat und Oleylmethacrylat im Gewichtsverhältnis 98/1/1, hergestellt durch Fällungspolymerisation in Cyclohexan
- Polymer 2:: Copolymer aus Methacrylsäure, Ethylacrylat und einem Methacrylester eines mit 25 Ethylenoxideinheiten umgesetzten C₁₆/C₁₈-Fettalkohols im Gewichtsverhältnis 60/83/8, hergestellt durch Emulsionspolymerisation in Wasser und anschließende Sprühtrocknung.

- Öl 1:: dünnflüssiges Paraffinöl nach Pharmakopöe Helvetika
- Öl 2:: Paraffinöl mit einer Viskosität (20°C) von 110 mPas
- Öl 3:: Isopropylmyristat
- Öl 4:: Sonnenblumenöl

### Beispiel 1

Polymer 1 wurde in einer Kompaktieranlage zu einem staubarmen Granulat verarbeitet. Dazu wurde das Pulver zunächst mit 20 Gew.-% von Öl 1 gemischt und anschließend aus einem Trichter mit Hilfe einer Stopfschnecke in den Spalt eines Walzenpaares (Walzendurchmesser = 200 mm, Walzenbreite = 50 mm) gefördert und zwischen den Walzen bei Drücken von ca. 80 MPa verpreßt. Die entstehende Schülpe wurde mit einem Fadenbrecher vorzerkleinert und mit einem Siebzerkleinerer auf 1,6 mm begrenzt. Das entstandene Granulat wurde bei 300 µm abgesiebt, das Feingut in den Vorlagetrichter zurückgeführt. Bei der Nutzfraktion handelte es sich um ein abriebarmes Produkt mit gegenüber dem Ausgangspulver deutlich erhöhter Schüttdichte, das trotz seiner Festigkeit eine gute Dispergierbarkeit in Ölphasen aufwies.

### Beispiele 2 bis 3

Die angegebenen Polymere wurden mit 20 Gew.-% an Öl 1 gemischt und auf einer Tablettenpresse bei Drücken von ca. 50 MPa verpreßt. Die entstandenen Tabletten (Durchmesser = 10 mm, Höhe = 1,5 mm) wurden analog Beispiel 1 zerkleinert und klassiert.
- Beispiel 2:: Polymer 1
- Beispiel 3:: Polymer 3

### Beispiel 4 bis 7

Polymer 1 wurde mit unterschiedlichen Mengen an Öl 1 gemischt und auf einer Tablettenpresse bei Drücken von ca. 50 MPa verpreßt. Die entstandenen Tabletten (Durchmesser = 10 mm, Höhe = 1,5 mm) wurden analog Beispiel 1 zerkleinert und klassiert.
- Beispiel 4:: 50 Gew.-%
- Beispiel 5:: 20 Gew.-%
- Beispiel 6:: 5 Gew.-%
- Beispiel 7:: 1 Gew.-%

### Verarbeitbarkeit in Öl

0,4 g des Kompaktats aus Beispiel 1 wurden in einem Becherglas in 30 ml Paraffinöl gegeben und auf einem Magnetrührer für eine Stunde gerührt. Nach Zugabe von 100 ml Wasser wurde eine weitere Stunde gerührt. Daraufhin wurde 4 ml einer 10 %igen Triethanolamin-Lösung in Wasser zugegeben und die Mischung mit einem Flügelrührer verrührt.

Man erhielt eine gleichmäßige Emulsion, die sich bei weiterem Stehen nicht veränderte und in ihren Eigenschaften einer Emulsion entsprach, die aus dem nicht-kompaktierten Polymer 1 unmittelbar erhalten wurde.

Nach Homogenisation mit einem Ultraturrax erhielt man eine weiße, feine Emulsion, die sich auch bei längerem Stehen nicht weiter veränderte.

In ähnlicher Weise erhielt man aus den Kompaktaten der Beispiele 2 bis 7 analoge Emulsionen, wobei in Beispiel 4 insgesamt 0,8 g des Kompaktats eingesetzt wurden.

Die Kompaktate der Beispiele 8 bis 11 wurden in analoger Weise und mit einem entsprechend guten Ergebnis wie Beispiel 1 in eine ölphase eingearbeitet. Beispiel 11 erforderte allerdings eine etwa verdoppelte Dispergierzeit im Paraffinöl, um eine quellkörperfreie Emulsion zu erhalten.

### Vergleichsbeispiel

In analoger Weise wie Beispiel 1 wurde ohne den Zusatz des Paraffinöls 1 ein Kompaktat aus Polymer 1 hergestellt. Das Produkt wurde in analoger Weise wie oben beschrieben zu einer Emulsion verarbeitet. Nach einer Stunde Rühren mit dem Magnetrührer waren die Granulate noch nicht oder erst sehr unvollkommen zerfallen. Nach Zugabe der Wasserphase erhielt man größere Gelbrocken, die sich nicht auflösten. Auch nach Basenzusatz und intensiver verrührung mit dem Flügelrührer blieben große Gelbrocken erhalten. Auch eine deutliche Verlängerung der Einrühr- und Dispergierzeiten brachte keine wesentliche Änderung. Durch Homogenisation erhielt man eine zunächst weiße Emulsion, in der jedoch klare, gequollene Gelbrocken vorhanden waren.

## Patentansprüche

1. Granulate, erhältlich durch verpressen von Mischungen aus mindestens einem pulverförmigen, rheologiemodifizierenden, carboxylgruppenhaltigen Polymer als Hauptkomponente und mindestens einer öllöslichen Komponente, wobei eine Polymerkomponente erhältlich durch radikalisch initiierte Copolymerisation von Monomergemischen aus
a) 50 bis 99,93 Gew.-% einer monoethylenisch ungesättigten C₃-C₆-Monocarbonsäure, einer monoethylenisch ungesättigten C₄-C₈-Dicarbonsäure oder deren Anhydride oder Salze oder Mischungen aus den genannten Carbonsäuren, deren Anhydriden und/oder Salzen,
b) 0,05 bis 5 Gew.-% einer oder mehrerer Verbindungen mit mindestens zwei ethylenisch ungesättigten, nicht konjugierten Doppelbindungen im Molekül als Vernetzer,
c) 0,02 bis 10 Gew.-% mindestens eines C₁-C₃₀-Alkyl(meth)acrylats und
d) 0 bis 50 Gew.-% anderen mit den Monomeren (a), (b) und (c) copolymerisierbaren wasserunlöslichen Monomeren verwendet wurde.

2. Granulate gemäß Anspruch 1, wobei eine Polymerkomponente, erhältlich durch radikalisch initiierte Copolymerisation von Monomergemischen aus
a) 85 bis 99,8 Gew.-% Acrylsäure oder Methacrylsäure oder 50 bis 64,8 Gew.-% Fumarsäure, Maleinsäure oder Maleinsäureanhydrid,
b) 0,1 bis 3 Gew.-% mindestens eines Allylethers von mehrwertigen Alkoholen mit mehr als einer Allyletherfunktion im Molekül oder Ester von monoethylenisch ungesättigten Carbonsäuren mit C₃-C₂₄-Alkoholen, Trivinylcyclohexan oder Divinylglycol,
c) 0,1 bis 5 Gew.-% mindestens eines C₈-C₂₄-Alkyl(meth)acrylats und
d) 0 - 5 Gew.-% (bei Acrylsäure oder Methacrylsäure als Monomerkomponente (a)) oder 35 - 50 Gew.-% (bei Fumarsäure, Maleinsäure oder Maleinsäureanhydrid) anderen mit den Monomeren (a), (b) und (c) copolymerisierbaren C₄-C₃₀-Alkenen verwendet wurde.

3. Granulate gemäß den Anspruch 1 oder 2, erhältlich durch Verpressen der Polymerpulver mit Wachsen, nichtionischen Emulgatoren, Fetten und/oder Ölen.

4. Granulate gemäß den Ansprüchen 1 bis 3, erhältlich durch Verpressen der Polymerpulver mit in der Kosmetik und/oder Pharmazie üblichen Ölen.

5. Granulate gemäß den Ansprüchen 1 bis 4, erhältlich durch Verpressen der Polymerpulver mit 0,1 bis 100 Gew.-% der öllöslichen Komponente bezogen auf das Polymergewicht.

6. Granulate gemäß den Ansprüchen 1 bis 5, erhältlich durch Verpressen der Polymerpulver mit 1 bis 50 Gew.-% der öllöslichen Komponente bezogen auf das Polymergewicht.

7. Granulate gemäß den Ansprüchen 1 bis 6, erhältlich durch Verprassen der Polymerpulver mit 2,5 bis 30 Gew.-% der öllöslichen Komponente bezogen auf das Polymergewicht.

8. Granulate gemäß den Ansprüchen 1 bis 7 mit einer mittleren Teilchengröße im Bereich von 20 µm bis 10 mm.

9. Verfahren zur Herstellung der Granulate gemäß den Ansprüchen 1 bis 8, **dadurch gekennzeichnet, daß** die carboxylhaltigen Polymerpulver in Gegenwart mindestens einer öllöslichen Komponente verpreßt werden.

10. Verwendung der Granulate gemäß den Ansprüchen 1 bis 8 in kosmetischen oder pharmazeutischen Zubereitungen.

11. Kosmetische oder pharmazeutische Zubereitungen, enthaltend Granulate gemäß den Ansprüchen 1 bis 8 und übliche Zusatzstoffe.

## Claims

1. A granular material obtainable by pressing mixtures of at least one pulverulent, rheology-modifying, carboxyl-containing polymer as main component and at least one oil-soluble component, wherein use is made of a polymer component obtainable by free-radical-initiated copolymerization of monomer mixtures comprising
a) from 50 to 99.93% by weight of a monoethylenically unsaturated C₃-C₆-monocarboxylic acid, a monoethylenically unsaturated C₄-C₈-dicarboxylic acid or their anhydrides or salts or mixtures of the specified carboxylic acids, their anhydrides and/or salts,
b) from 0.05 to 5% by weight of one or more compounds having at least two ethylenically unsaturated, nonconjugated double bonds in the molecule as crosslinker,
c) from 0.02 to 10% by weight of at least one C₁-C₃₀-alkyl (meth)acrylate and
d) from 0 to 50% by weight of other water-insoluble monomers copolymerizable with the monomers (a), (b) and (c).

2. A granular material as claimed in claim 1, wherein use is made of a polymer component obtainable by free-radical-initiated copolymerization of monomer mixtures comprising
a) from 85 to 99.8% by weight of acrylic acid or methacrylic acid or from 50 to 64.8% by weight of fumaric acid, maleic acid or maleic anhydride,
b) from 0.1 to 3% by weight of at least one allyl ether of polyhydric alcohols having more than one allyl ether funtion in the molecule or esters of monoethylenically unsaturated carboxylic acids with C₃-C₂₄-alcohols, trivinylcyclohexane or divinyl glycol,
c) from 0.1 to 5% by weight of at least one C₈-C₂₄-alkyl (meth)acrylate and
d) 0 - 5% by weight (in the case of acrylic acid or methacrylic acid as monomer component (a)) or 35 - 50% by weight (in the case of fumaric acid, maleic acid or maleic anhydride) of other C₄-C₃₀-alkenes copolymerizable with the monomers (a), (b) and (c).

3. A granular material as claimed in claim 1 or 2 obtainable by pressing the polymer powders with waxes, nonionic emulsifiers, fats and/or oils.

4. A granular material as claimed in any of claims 1 to 3 obtainable by pressing the polymer powders with oils customary in cosmetics and/or pharmacy.

5. A granular material as claimed in any of claims 1 to 4 obtainable by pressing the polymer powders with from 0.1 to 100% by weight of the oil-soluble component, based on the polymer weight.

6. A granular material as claimed in any of claims 1 to 5 obtainable by pressing the polymer powders with from 1 to 50% by weight of the oil-soluble component, based on the polymer weight.

7. A granular material as claimed in any of claims 1 to 6 obtainable by pressing the polymer powders with from 2.5 to 30% by weight of the oil-soluble component, based on the polymer weight.

8. A granular material as claimed in any of claims 1 to 7 having a mean particle size in the range from 20 µm to 10 mm.

9. A process for producing a granular material as claimed in any of claims 1 to 8, which comprises pressing the carboxyl-containing polymer powders in the presence of at least one oil-soluble component.

10. Use of a granular material as claimed in any of claims 1 to 8 in cosmetic or pharmaceutical preparations.

11. A cosmetic or pharmaceutical preparation comprising a granular material as claimed in any of claims 1 to 8 and customary additives.

## Revendications

1. Granulés, pouvant être obtenus par compression de mélanges d'au moins un polymère pulvérulent, modificateur de rhéologie, contenant des groupes carboxyles, en tant que composant principal, et d'au moins un composant soluble dans l'huile, tandis qu'on a utilisé un composant polymère pouvant être obtenu par copolymérisation initiée par des radicaux libres de mélanges de monomères de
a) 50 à 99,93 % en poids d'un acide mono-carboxylique en C₃-C₆ à insaturation monoéthylénique, un acide dicarboxylique en C₄-C₈ à insaturation monoéthylénique ou leurs anhydrides ou des sels ou mélanges des acides carboxyliques indiqués, leurs anhydrides et/ou leurs sels,
b) 0,05 à 5 % en poids d'un ou plusieurs composés ayant au moins deux doubles liaisons éthyléniquement insaturées, non conjuguées dans la molécule, comme agent de réticulation,
c) 0,02 à 10 % en poids d'au moins un (méth)acrylate d'alkyle en C₁-C₃₀ et
d) 0 à 50 % en poids d'autres monomères insolubles dans l'eau, copolymérisables avec les monomères (a), (b) et (c) .

2. Granulés selon la revendication 1, dans lesquels on a utilisé un composant polymère pouvant être obtenu par copolymérisation initiée par des radicaux libres de mélanges de monomères de
a) 85 à 99,8 % en poids d'acide acrylique ou d'acide méthacrylique, ou 50 à 64,8 % en poids d'acide fumarique, d'acide maléique ou d'anhydride d'acide maléique,
b) 0,1 à 3 % en poids d'au moins un éther allylique d'alcools polyvalents ayant plus d'une fonction éther d'allyle dans la molécule ou d'esters d'acides carboxyliques à insaturation monoéthylnique avec des alcools en C₃-C₂₄, de trivinylcyclohexane ou de divinylglycol,
c) 0,1 à 5 % en poids d'au moins un (méth)acrylate d'alkyle en C₈-C₂₄ et
d) 0 à 5 % en poids (pour l'acide acrylique ou l'acide méthacrylique en tant que composant monomère (a)) ou 35 - 50 % en poids (pour l'acide fumarique, l'acide maléique ou l'anhydride d'acide maléique) d'autres alcènes en C₄-C₃₀ copolymérisables avec les monomères (a), (b) et (c) .

3. Granulés selon la revendication 1 ou 2, pouvant être obtenus par compression des poudres de polymère avec des cires, des émulsifiants non-ioniques, des graisses et/ou des huiles.

4. Granulés selon les revendications 1 à 3, pouvant être obtenus par compression des poudres de polymère avec des huiles usuelles en cosmétique et/ou en pharmacie.

5. Granulés selon les revendications 1 à 4, pouvant être obtenus par compression des poudres de polymère avec 0,1 à 100 % en poids des composants solubles dans l'huile par rapport au poids de polymère.

6. Granulés selon les revendications 1 à 5, pouvant être obtenus par compression des poudres de polymère avec 1 à 50 % en poids des composants solubles dans l'huile par rapport au poids de polymère.

7. Granulés selon les revendications 1 à 6, pouvant être obtenus par compression des poudres de polymère avec 2,5 à 30 % en poids des composants solubles dans l'huile par rapport au poids de polymère.

8. Granulés selon les revendications 1 à 7 ayant une taille moyenne des particules dans l'intervalle de 20 µm à 10 nm.

9. Procédé pour la préparation des granulés selon les revendications 1 à 8, **caractérisé par le fait que** les poudres de polymère contenant des carboxyles sont comprimées en présence d'au moins un composant soluble dans l'huile.

10. Utilisation des granulés selon les revendications 1 à 8 dans des préparations cosmétiques ou pharmaceutiques.

11. Préparations cosmétiques ou pharmaceutiques, contenant des granulés selon les revendications 1 à 8 et des additifs usuels.
